# EUROPEAN PATENT APPLICATION

(11) **EP 1 864 657 A1**
(43) Date of publication of application: **12.12.2007**
(21) Application number: 07109867.7
(22) Date of filing: 08.06.2007
(51) Int. Cl.: A61K 31/23, A61P 17/00, A61P 17/06, A61P 17/08, A61P 17/04, A61P 17/02, A61P 17/10, A61P 17/14, A61P 17/16

(54) **Treatment of cutaneous neurogenic inflammation**

(30) Priority: 08.06.2006 DK 200600776
(71) Applicant: Astion Pharma A/S, 2100 Copenhagen Ø (DK); Astion Development A/S, 2100 Copenhagen O (DK)
(72) Inventor: Weidner, Morten Sloth, 2830 Virum (DK)
(74) Representative: Plougmann & Vingtoft A/S

(57) **Abstract**

The glyceryl fatty acid monoester of octanoic acid, i.e. 1-glyceryl monocaprylate has, unlike other glyceryl fatty acid esters, such as 1-glyceryl monocaprate, been shown to inhibit cutaneous neurogenic inflammation. Therefore, this invention features uses and dermatological compositions of 1-glyceryl monocaprylate or analogues thereof for the treatment of cutaneous neurogenic inflammation, such as pruritus, pruritic skin disorders and seborrheic dermatitis, even without the need of co-administering other antiinflammatory agents.

## Description

### FIELD OF THE INVENTION

The invention provides uses of 1-glyceryl monocaprylate as the therapeutically active agent for the treatment of skin disorders or skin disorders characterised by or associated with cutaneous neurogenic inflammation.

### BACKGROUND OF THE INVENTION

A number of skin conditions and skin disorders are known to have cutaneous neurogenic inflammation as part of their pathogenesis. Particularly, it can be mentioned that skin conditions and skin disorders associated with cutaneous neurogenic inflammation often are triggered or exacerbated by stress *(*Katsarou-Katsari A, Filippou A, Theoharides TC. Effect of stress and other psychological factors on the pathophysiology and treatment of dermatoses. Int J Immunopathol Pharmacol 1999 Jan;12(1):7-11*).* The nervous system of the skin and its interaction with dermal cells, e.g. keratinocytes and infiltrating leucocytes is relatively well understood and currently under extensive investigation. Afferent somatic nerves with fine unmyelinated (C-) or myelinated (Aδ-) fibers derive from dorsal root ganglia and innervate the skin. Both types of fibers respond to a range of physiologic stimuli such as physical trauma (heat, cold, nociception, mechanical distension, and UV light) and even low-intensity mechanical stimulation. Furthermore, specialized nociceptor fibers can be activated by a wide range of chemicals, which indicates that not only physical but also chemical stimuli are capable of activating the cutaneous nervous system. On stimulation, the nerves rapidly release active neuropeptides such as substance P into the microenvironment.

The most well described dermal neuropeptide is substance P, which is a powerful mediator of inflammatory events in the skin. Substance P stimulates release of tumor necrosis factor *α, histamine, prostaglandin* D₂, and leukotriene B₄ from skin mast cells *(*Furutani K, Koro O, Hide M, Yamamoto S. Substance P- and antigen-induced release of leukotriene B4, prostaglandin D2 and histamine from guinea pig skin by different mechanisms in vitro. Arch Dermatol Res 1999 Jul;291(7-8):466-73*).* Furthermore it stimulates keratinocytes to produce the pro-inflammatory cytokines IL-1α, IL-1β, and IL-8 *(*Song IS, Bunnett NW, Olerud JE, Harten B, Steinhoff M, Brown JR, et al. Substance P induction of murine keratinocyte PAM 212 interleukin 1 production is mediated by the neurokinin 2 receptor (NK-2R). Exp Dermatol 2000 Feb;9(1):42-52*).* In addition substance P is capable of activating human dermal microvascularendothelial cells in vivo and in vitro.

Thus, substance P induces up-regulation of cell adhesion molecules such as P-selectin, intercellular adhesion molecule (ICAM-1), and vascular cell adhesion molecule (VCAM-1) *(*Quinlan KL, Naik SM, Cannon G, Armstrong CA, Bunnett NW, Ansel JC, et al. Substance P activates coincident NF-AT- and NF-kappa B-dependent adhesion molecule gene expression in microvascular endothelial cells through intracellular calcium mobilization. J Immunol 1999 Nov 15;163(10):5656-65*);* recruitment of neutrophils and eosinophils; and the release of chemokines such as IL-8.

Substance P is released together with other neuropeptides upon activation of TRP vanilloid receptor 1 (TRPV-1). Low pH levels that accompany inflammatory responses can increase the response of TRPV-1 to noxious stimuli, which suggests that the response of sensory nerve fibers during neurogenic inflammation results, at least in part, from activation of vanilloid receptors through an excess of protons, eicosanoid ligands especially leukotriene B4, and probably bradykinin *(*Carr MJ, Hunter DD, Jacoby DB, Undem BJ. Expression of tachykinins in nonnociceptive vagal afferent neurons during respiratory viral infection in guinea pigs. Am J Respir Crit Care Med 2002 Apr 15;165(8):1071-5*).* Recent data support a central role of TRPV-1 in neurogenic inflammation *(*Rigoni M, Trevisani M, Gazzieri D, Nadaletto R, Tognetto M, Creminon C, et al. Neurogenic responses mediated by vanilloid receptor-1 (TRPV1) are blocked by the high affinity antagonist, iodo-resiniferatoxin. BrJ Pharmacol 2003 Mar;138(5):977-85*).* For example various lipids including sebum components and their degradation products are able to directly activate TRPs *(*Hu HZ, Xiao R, Wang C, Gao N, Colton CK, Wood JD, et al. Potentiation of TRPV3 channel function by unsaturated fatty acids. J Cell Physiol 2006 Jul; 208(1): 201-12*).*

Therefore, depletion of substance P and related neuropeptides seems to be a promising target in preventing many of the neuro-inflammatory events seen in a number of skin disorders.

Moreover, endocannabinoids may be implicated in mediating cutaneous neurogenic inflammation. Endocannabinoids are fatty acid derivatives produced by the modification of membrane fatty acids, in particular arachidonic acid, and have varying specificities for the two known cannabinoid receptors CB1 and CB2. Arachidonoylethanolamide (AEA; previously known as anandamide) is an endogenous fatty acid amide that are degraded by the enzyme Fatty Acid Amide Hydrolase (FAAH). Cannabinoid binding in immune cells generally inhibits the production of cytokines during innate and adaptive immune responses, both in animal models and in humans in vitro and in vivo *(*Klein TW, Friedman H, Specter S. Marijuana, immunity and infection. J Neuroimmunol 1998 Mar 15;83(1-2):102-15*.).* In the skin, expression of CB1 and CB2 has been detected in neurons, keratinocytes, cutaneous mast cells, and macrophages *(*Stander S, Schmelz M, Metze D, Luger T, Rukwied R. Distribution of cannabinoid receptor 1 (CB1) and 2 (CB2) on sensory nerve fibers and adnexal structures in human skin. J Dermatol Sci 2005 Jun;38(3):177-88*)* and peripherally administered cannabinoids have shown to induce anti-inflammatory, antinociceptive and antihyperalgestic effects both in humans *(*Coutaux A, Adam F, Willer JC, Le BD. Hyperalgesia and allodynia: peripheral mechanisms. Joint Bone Spine 2005 Oct;72(5):359-71*).* FAAH is induced in cutaneous inflammation and inhibition of FAAH may increase the endogenous levels of AEA leading to an anti-inflammatory effect.

Therefore, the inhibition of FAAH is an attractive therapeutic strategy in cutaneous inflammation by enhancing the levels of anti-inflammatory endocannabinoids *(*Maccarrone M, Di RM, Battista N, Gasperi V, Guerrieri P, Rossi A, et al. The endocannabinoid system in human keratinocytes. Evidence that anandamide inhibits epidermal differentiation through CB1 receptor-dependent inhibition of protein kinase C, activation protein-1, and transglutaminase. J Biol Chem 2003 Sep 5;278(36):33896-903*).*

The object of this invention is to provide new safe medication in the treatment of cutaneous neurogenic inflammation.

Glyceryl fatty acid esters or analogues thereof are generally considered as compounds with anti-microbial properties and not other pharmacologically relevant activities. For instance the patent application US2006229364A of 3M relates to glyceryl fatty acid esters for use as antiviral lipids, the patent application US2004072705A of Dow pharmaceuticals relates to antiseptic skin cleansers comprising a glyceryl fatty acid ester, the patent application W09820872A of Lipomedica relates to the use of monoglycerides for treating mucosa infections. Moreover, the patent application WO9010441 relates to fatty acid esters (monocarboxylic acids) or amines having antimicrobial activity. Such esters may be used in the treatment of non-infectious inflammatory dermatological diseases in which microbes play a significant adjunctive pathophysiological role. WO9010441 specifically teaches that fatty acids of chain lengths of 9 or higher (e.g. oleic acid) and esters and amides thereof can be used in the treatment of seborrheic dermatitis, psoriasis and acne. However, WO9010441 is completely silent with respect to glyceryl monocaprylate.

The patent application WO 2004/000333 (Astion Development) teaches that combinations of glyceryl fatty acid esters and pyridine carboxy derivatives, such as niacinamide, are effective, but only in combination, in the treatment of inflammatory diseases. As shown in WO 2004/000333, only the combined administration of 1-glyceryl monocaprylate and niacinamide (molar ratio 2:7) showed inhibitory effect on acute inflammation with statistically significant effect (See Example 111). Further data in the same application shows that the topical administration of a 5% gel composition of 1-glyceryl monocaprylate and niacinamide (molar ratio 2:7) to skin of patients with seborrheic dermatitis resulted in pronounced improvement of the disease.

### SUMMARY OF THE INVENTION

It has now been found, for the first time, that 1-glyceryl monocaprylate (GMCY) exhibits anti-inflammatory activity of its own and specifically modifies biochemical signalling pathways involved in cutaneous neurogenic inflammation.

Accordingly, a first aspect of the invention relates to uses of 1-glyceryl monocaprylate or an analogue thereof, such as dermatological compositions comprising 1-glyceryl monocaprylate or an analogue thereof in the treatment of cutaneous neurogenic inflammation, such as in the treatment of a skin condition or skin disorder associated with cutaneous neurogenic inflammation or in the treatment of cutaneous neurogenic inflammation in a subject diagnosed with a skin disorder.

In another wording, this first aspect relates to use of 1-glyceryl monocaprylate or an analogue thereof for the preparation of a dermatological composition in the treatment of cutaneous neurogenic inflammation, such as in the treatment of a skin condition or skin disorder associated with cutaneous neurogenic inflammation or in the treatment of cutaneous neurogenic inflammation in a subject diagnosed with a skin disorder.

A second aspect of the invention features a dermatological composition formulated for the application to skin for the local treatment of a skin disorder, the dermatological composition comprises 1-glyceryl monocaprylate or an analogue thereof as the therapeutically active ingredient and further comprising a dermatologically acceptable vehicle.

### DETAILED DESCRIPTION OF THE INVENTION

It has surprisingly been found that GMCY is an anti-inflammatory agent on its own, i.e. without the requirement of being combined with another drug agent as for example niacinamide. The present inventor has specifically shown that GMCY, but not the 1-glyceryl monocaprate, inhibits capsaicin-induced neurogenic inflammation in mice (Example 1). Capsaicin induces inflammation via activation of the vanilloid receptor (TRPV-1) and the co-administration of GMCY was shown to result in less inflammation following repeated administration of capsaicin. It was demonstrated that GMCY did not bind to the TRPV-1 receptor, thus indicating that GMCY most probably prevents cutaneous neurogenic inflammation through depletion of the pool of neuropeptides, such as Substance P, that usually are released by activation of TRPV-1.

Furthermore, it has been demonstrated that GMCY directly inhibits the enzyme fatty acid amide hydrolase (FAAH) (see Example 2).

GMCY has also been shown to dose dependently inhibit bacterial lipopolysaccharide (LPS) induced release of TNF-α and IL-1β from human peripheral blood mononuclear cells (PBMC). Pro-inflammatory cytokines like TNF-α and IL-1 play a central role in initiating and potentiating the inflammatory response in inflammatory skin disorders. They are expressed early in the inflammatory response and the effects include local changes, resulting in mobilization of antigen presenting cells and activation of endothelial cells to express adhesion molecules.

Finally, it has been found that 1-glyceryl monocaprylate inhibits protein tyrosine kinase SYK.

Fortunately, unlike other glyceryl fatty acid esters of higher chain lengths, GMCY does not activate peroxisome proliferator-activated receptors (PPARs) (see Example 4), because such activation would result in stimulation of lipid biosynthesis in both sebocytes and keratinocytes and may contribute to sebocyte differentiation and formation and to keratinocyte growth and differentiation *(see* Rosenfield RL, Deplewski D, Greene ME. Peroxisome proliferator-activated receptors and skin development. Horm Res 2000;54(5-6):269-749*)*. Therefore, glyceryl fatty acid esters like glyceryl monocaprate and glyceryl monolaurin and other glyceryl fatty acid esters of higher chain lengths are in contrary to GMCY unsuitable for use in the treatment of diseases with high sebum or keratinocyte activity like seborrheic dermatitis, psoriasis and acne.

Together, these results support the view that GMCY, and not other glyceryl fatty acid esters with higher carbon chain length of the acid, is a promising new drug agent for use in the treatment of cutaneous neurogenic inflammation - even without simultaneous co-administering of other drug agents. Notably, previous studies attempting to show effect of GMCY in preventing acute inflammation in mice could has failed in that any effect could not be proven statistically *(See Example 111 of* WO 2004/000333*).* Furthermore, it is well recognized that activity of a compound in inhibiting cellular mediated inflammation is not predictive of activity to inhibit neurogenic inflammation or itch *(see* U.S. Pat. No.6,090,811 *or* Inoue H, Nagata N, Koshihara Y. Profile of capsaicin-induced mouse ear oedema as neurogenic inflammatory model: comparison with arachidonic acid-induced ear oedema. Br J Pharmacol 1993 Dec; 110(4):1614-20*).* The irritation response may be due to the direct effect on the skin of certain topical product chemicals (e.g. capsaicin), a response by the immune system directed toward the chemicals alone or in combination with skin components (e.g. sebum), or to a skin disorder or disorder.

Therefore, a first aspect of the invention relates to uses of 1-glyceryl monocaprylate or an analogue thereof, such as to uses of a dermatological composition comprising 1-glyceryl monocaprylate or an analogue thereof, in the treatment of cutaneous neurogenic inflammation, such as in the treatment of a skin condition or skin disorder associated with cutaneous neurogenic inflammation, such as in the treatment of cutaneous neurogenic inflammation in a subject suffering from or diagnosed with a skin condition or skin disorder. In another wording, this first aspect relates to use of 1-glyceryl monocaprylate or an analogue thereof for the preparation of a dermatological composition in the treatment of cutaneous neurogenic inflammation, such as in the treatment of a skin condition or skin disorder associated with cutaneous neurogenic inflammation or in the treatment of cutaneous neurogenic inflammation in a subject diagnosed with a skin disorder.

As used herein, the term "cutaneous neurogenic inflammation" also referred to as "neuroinflammation in skin" is meant to define those types of inflammation triggered by sensory nerve activation in skin. Activation of small diameter sensory neurons induces release of inflammatory neuropeptides such as substance P and calcitonin gene-related peptide. These substances, in turn, act on peripheral blood vessels and immune cells producing an inflammatory response that is characterized by erythema, edema, warmth and hypersensitivity *(*Richardson JD, Vasko MR. Cellular mechanisms of neurogenic inflammation. J Pharmacol Exp Ther 2002 Sep;302(3):839-45*).* In mouse skin, an edema response occurs rapidly upon application of a vanilloid receptor activator, such as capsaicin or resiniferatoxin. Therefore, cutaneous neurogenic inflammation can be defined as cutaneous inflammation provoked, caused by, or mediated by the activation of a vanilloid receptor or by the application of capsaicin or resiniferatoxin to skin. Notably, non-neurogenic inflammation involves the release of inflammatory substances (e.g. histamines, prostaglandins, cytokines, leukotrienes, etc) from the blood vessels and connective tissue.

The phrase "a subject in need thereof" is meant to define a human or an animal suffering from or diagnosed with a skin condition or skin disorder described herein.

The term "a dermatological composition" is meant to define a pharmaceutical composition or a cosmeceutical composition formulated for cutaneous administration, such as formulated for the application to a portion of skin affected by a skin condition or skin disorder, such as a skin condition or skin disorder associated with cutaneous neurogenic inflammation. Thus, the term "dermatological composition" does not include compositions formulated for or intended for transdermal, subcutaneous or percutaneous administration or for being administered as an implant.

The term "skin" is meant to include, but not limited to, the skin of the scalp,face, ears, eyelid, trunk, arm, legs, feeds and hands.

The phrase "1-glyceryl monocaprylate or an analogue thereof" is meant to define that the therapeutically active ingredient may be 1-glyceryl monocaprylate or a substance with a molecular structure closely resembling 1-glyceryl monocaprylate. Currently, it is considered that an analogue of 1-glyceryl monocaprylate is meant to include other esters of caprylic acid and polyhydric alcohols with a carbon chain length of 2 to 4 carbon atoms, such as monoesters of caprylic acid and 1,2-propylene glycol, 1,3-propylene glycol, 1,2 butanediol, 1,3 butanediol, 1,4 butanediol, and 2,3 butanediol where the ester preferably is positioned on carbon atom 1 of the polyhydric alcohol. More specifically, such esters of interest are 1-propylene glycol monocaprylate and 1-butanediol monocaprylate.

The 1-glyceryl monocaprylate or an analogue thereof may be obtained synthetically or extracted or derived from a natural source like coconut oil according to methods well-known in the art. The synthesized or extracted 1-glyceryl monocaprylate or an analogue thereof may contain related glyceryl esters in the form of caprylate diesters or 2-glyceryl monocaprylates or smaller amounts of glyceryl esters of higher acid carbon chain length. The purity of 1-glyceryl monocaprylate may be at least 80% by weight, preferably at least 85%, such as at least 90% by weight, with respect to the content of 1-glyceryl monocaprylate. That is to say that less than 20% by weight, such as preferably less than 15% by weight of the 1-glyceryl monocaprylate material consists of glyceryl dicaprylate and/or a 2-glyceryl monocaprylate.

A number of skin conditions and skin disorders are known to have cutaneous neurogenic inflammation as part of their pathogenesis. Particularly, it can be mentioned that skin conditions and skin disorders associated with cutaneous neurogenic inflammation often are triggered or exacerbated by stress.

Therefore, uses described herein include the treatment of stress-induced skin conditions or skin disorders.

Typically examples on skin conditions or skin disorders associated with cutaneous neurogenic inflammation are the following:

### Pruritus and pruritic skin disorders

Pruritus can be caused by a number of disease states though still be mediated by release of neuropeptides, such as substance P from dermal neurons. Thus, pruritus is caused by neurogenic inflammation *(see* Steinhoff M, Stander S, Seeliger S, Ansel JC, Schmelz M, Luger T. Modern aspects of cutaneous neurogenic inflammation. Arch Dermatol 2003 Nov; 139(11):1479-88*).* Pruritus, such as neurogenic pruritus, may be a symptom associated with systemic diseases like endocrine disease, such as diabetes mellitus, hyperthyroidism, hypothyroidism, and thyrotoxicosis; kidney failure or renal failure or dysfunction (e.g. uraemic itching); liver failure or dysfunction, such as cholestasis and primary biliary cirrhosis; Haematological diseases, such as polycythaemia vera, Hodgkin's Disease,allergy, such as food allergy, and cancers like leukaemia, brain tumour, adenocarcinoma and squamous cell carcinoma, where treatment with anti-histamines fails. Moreover pruritus is presented in pruritic skin diseases. Typically examples are, but not limited to, acne, alopecia, anogenital pruritus, aquagenic pruritus, atopic dermatitis, chickenpox infection, dermatitis herpetiformis, dry skin, hemorrhoids, insect sting or bites, lichen simplex, photodermatitis, photosensitivity, pityriasis rosea, pregnancy-related dermatoses, prurigo nodularis, prurigo planus, pruritus ani, pruritic otitis, pruritic papular eruption of HIV, psoriasis, ringworm, scabies, seborrheic dermatitis, senile pruritus, shingles, urticaria, wounds and sunburn.

### Seborrheic dermatitis

Seborrheic dermatitis is an inflammatory skin disorder affecting the upper layers of the skin, dermis and stratum corneum. The pathogenesis of seborrheic dermatitis is complex and at present not fully elucidated. It is currently considered that factors like sebaceous gland secretions, microfloral metabolism of Malassezia yeast, and individual susceptibility and immune responses are mostly relevant factors to consider as relevant in the pathogenesis *(See* Gupta AK, Bluhm R. Seborrheic dermatitis. J Eur Acad Dermatol Venereol 2004 Jan;18(1):13-26*).* Specifically, it has been demonstrated that Malassezia in the skin induces an irritant non-immunogenic stimulation of the immune system with release of inflammatory cytokines like IL-1 and TNFα in patients with seborrheic dermatitis *(*Faergemann J, Bergbrant IM, Dohse M, Scott A, Westgate G. Seborrhoeic dermatitis and Pityrosporum (Malassezia) folliculitis: characterization of inflammatory cells and mediators in the skin by immuno-histochemistry. Br J Dermatol 2001 Mar;144(3):549-56*).* However a growing body of evidence supports the notion that the pathophysiology of seborrheic dermatitis has a strong and potentially causative neurogenic inflammatory aspect. This is supported by its association with a number of neurological disorders including parkinsonism, multiple sclerosis, postcerebrovascular accidents, epilepsy, central nervous system trauma, facial nerve palsy, and syringomyelia induced by neuroleptic drugs with extrapyramidal effects. Such conditions are known to be associated with neurogenic inflammation and notably seborrheic dermatitis occurs more frequently among sufferers of such conditions as compared to the general population. Another clear indication of a causative neurogenic inflammatory aspect of seborrheic dermatitis is the clinical observation that emotional stress and physical stress (e.g. exposure to cold or hot conditions) may cause significant aggravation of the clinical symptoms. Such factors are known to stimulate neurogenic inflammation through TRP vanilloid receptors. Furthermore, there is an important link between excess sebum secretion and degradation in seborrheic dermatitis and the development of neurogenic inflammation. The degradation of sebum is promoted by Malassezia species found in normal dimorphic human flora. 6-8 Yeasts of this genus predominate and are found in seborrheic regions of the body that are rich in sebaceous lipids (e.g., head, trunk, upper back). A causal role of Malassezia has been implied in seborrheic dermatitis, but a limited treatment response to antifungal agents and the fact that Malassezia generally occurs to the same extent in healthy individual indicates that the yeast plays a more secondary role in the pathogenesis of seborrheic dermatitis. It is expected that substance P and related neuropeptides are capable of inducing many of the inflammatory events seen in seborrheic dermatitis. For example various lipids including sebum components and their degradation products are able to directly activate TRPs. This may be an important link between excess sebum secretion and degradation in seborrheic dermatitis and the development of neurogenic inflammation.

### Alopecia areata

Alopecia areata is a recurrent nonscarring type of hair loss that can affect any hair-bearing area and is suggested to be associated with cutaneous neurogenic inflammation (*see* Zegarska B, Lelinska A, Tyrakowski T. Clinical and experimental aspects of cutaneous neurogenic inflammation. Pharmacol Rep 2006 Jan;58(1):13-21*).*

### Atopic dermatitis

Atopic dermatitis is a chronic, highly pruritic, eczematous skin disorder, which may be exacerbated by social, environmental, and biological triggers *(See* Huang CH, Kuo IC, Xu H, Lee YS, Chua KY. Mite allergen induces allergic dermatitis with concomitant neurogenic inflammation in mouse. I Invest Dermatol 2003 Aug;121 (2): 289-93 *and* Zegarska B, Lelinska A, Tyrakowski T. Clinical and experimental aspects of cutaneous neurogenic inflammation. Pharmacol Rep 2006 Jan;58(1):13-21*).*

### Lichen simplex

This is a skin disorder that is also named neurodermatitis - and defines a skin disorder characterised by chronic itching. The skin may become leathery and brownish in the affected area. This disorder may be associated with atopic dermatitis (eczema) or psoriasis. It may also be associated with nervousness, anxiety, depression, and other psychological disorders.

### Photodermatoses

Photodermatoses is a skin condition which develops following exposure to the sun and has skin rash as the predominant manifestation. Polymorphous Light Eruption is the most common type of photodermatoses. Another photodermatoses is actinic prurigo that is a chronic, pruritic skin disorder caused by an abnormal reaction to sunlight. Photodermatoses is thought to involve cutaneous neurogenic inflammation *(see* Zegarska B, Lelinska A, Tyrakowski T. Clinical and experimental aspects of cutaneous neurogenic inflammation. Pharmacol Rep 2006 Jan;58(1):13-21*).*

### Prurigo nodularis

Prurigo nodularis is a chronic condition characterized by a papulonodular eruption, pruritus and neurogenic inflammation *(*Lee MR, Shumack S. Prurigo nodularis: a review. Australas J Dermatol 2005 Nov;46(4):211-8*).*

### Psoriasis

Psoriasis is an immune-mediated disease, which affects the skin and joints. In psoriatic lesions an upregulation of neuropeptides such as substance P (SP), vasoactive intestinal peptide and calcitonin gene-related peptide (CGRP) along with marked proliferation of terminal cutaneous nerves have been reported *(see* Zegarska B, Lelinska A, Tyrakowski T. Clinical and experimental aspects of cutaneous neurogenic inflammation. Pharmacol Rep 2006 Jan;58(1):13-21*).*

### Rosacea

Rosacea is a common facial skin disease with symptoms of blushing, redness, telangiectasis, papules, pustules, and diffuse swelling of the skin. A neurogenic component of the disease is suggested involvind release of neuropeptides *(*Lonne-Rahm S, Nordlind K, Edstrom DW, Ros AM, Berg M. Laser treatment of rosacea: a pathoetiological study. Arch Dermatol 2004 Nov;140(11):1345-9 *and* Holzer P. Neurogenic vasodilatation and plasma leakage in the skin. Gen Pharmacol 1998 Jan;30(1):5-11*).*

### Shingles

Shingels is a skin rash caused by the same virus that causes chickenpox. The virus responsible for these conditions is called Varicella zoster and may cause post-herpetic neuralgia. This rash is known to be caused by substance P release and can be treated by capsaicin *(*Rains C, Bryson HM. Topical capsaicin. A review of its pharmacological properties and therapeutic potential in post-herpetic neuralgia, diabetic neuropathy and osteoarthritis. Drugs Aging 1995 Oct;7(4):317-28*).*

### Sunburn

Pain, associated with sunburn, involves stimulation of nocioceptive C-type fibers in the epidermis, which release substance P when activated *(*Brogden KA, Guthmiller JM, Salzet M, Zasloff M. The nervous system and innate immunity: the neuropeptide connection. Nat Immunol 2005 Jun; 6 (6):558-64*).* Sunburn is an intense, delayed, transient inflammatory response caused by acute overexposure to ultraviolet radiation (UVR) in sunlight, primarily ultraviolet B (UV-B).

### Urticaria

Urticaria is commonly known as hives. It consists of circumscribed areas of raised erythema and edema of the superficial dermis. Urticaria occurs following release of histamine, bradykinin, kallikrein, and other vasoactive substances from mast cells and basophils, resulting in intradermal edema from capillary and venous vasodilation and occasionally from leukocyte infiltration. In accordance with this invention, the type of urticaria to be treated is preferably bradykinin, such as substance P mediated urticaria, such as cold and heat induced urticaria. (see Holzer P. Neurogenic vasodilatation and plasma leakage in the skin. Gen Pharmacol 1998 Jan;30(1):5-11 *and* Miyazaki Y, Satoh T, Nishioka K, Yokozeki H. STAT-6-mediated control of P-selectin by substance P and interleukin-4 in human dermal endothelial cells. Am J Pathol 2006 Aug;169(2): 697-707*).*

### Cutaneous warts

Warts are benign proliferations of skin and mucosa caused by the human papilloma virus (HPV). Various types of warts include common wart, flat wart, filiform wart, plantar wart, mosaic wart. The sensitivity of a person to be infected by human papilloma virus and to present warts is thought to involve neurogenic inflammation.

In accordance herewith, one embodiment of the invention features uses and dermatological compositions comprising GMCY or an analogue thereof for the treatment of pruritus, such as specifically neurogenic pruritus. More specifically, some embodiments features the treatment of pruritus include the treatment of pruritus in a subject suffering from or diagnosed with diabetes mellitus, hyperthyroidism, hypothyroidism, thyrotoxicosis, kidney failure, uraemic itching, liver cholestasis, primary biliary cirrhosis, polycythaemia vera, Hodgkin's disease, food allergy and leukaemia, brain tumour and adenocarcinoma. Thus, embodiments herein features treatment of pruritus that is caused by or associated with a systemic disease selected from the group consisting of diabetes mellitus, hyperthyroidism, hypothyroidism, thyrotoxicosis, kidney failure, uraemic itching, liver cholestasis, primary biliary cirrhosis, polycythaemia vera, Hodgkin's disease, food allergy, leukaemia, brain tumour and adenocarcinoma.
Still more specifically, some embodiments of the invention features the treatment of a pruritic skin disorder, such as one selected from the group consisting of acne, alopecia, anogenital pruritus, aquagenic pruritus, atopic dermatitis, chickenpox infection, dermatitis herpetiformis, dry skin, hemorrhoids, insect sting or bites, lichen simplex, photodermatitis, photosensitivity, pityriasis rosea, pregnancy-related dermatoses, prurigo nodularis, prurigo planus, pruritus ani, pruritic otitis, pruritic papular eruption of HIV, psoriasis, ringworm, scabies, seborrheic dermatitis, senile pruritus, shingles, urticaria, wounds and sunburn.

Preferably, such embodiments are directed to the treatment of treatment of a pruritic skin disorder selected from the group consisting of atopic dermatitis, chickenpox infection, photodermatitis, photosensitivity, prurigo nodularis, prurigo planus, pruritus ani, pruritic otitis, ringworm, scabies, senile pruritus, shingles, urticaria, wounds and sunburn.

As may be understood, some skin disorders may present cutaneous neurogenic inflammation in addition to inflammation, and even some skin disorders can be treated effectively with agents targeting cutaneous neurogenic inflammation and by preventing the neurogenic inflammation, the inflammation is also prevented.

Therefore, one embodiment of the invention features uses and dermatological compositions comprising GMCY or an analogue thereof for the treatment of seborrheic dermatitis, including mild seborrheic dermatitis, moderate seborrheic dermatitis or severe seborrheic dermatitis. Typically, the lesions of seborrheic dermatitis are red (inflamed appearance) and scaly and even more typically, the skin suffers from intense itching. Seborrheic dermatitis may affect any hair-bearing skin area or any area of the skin with sebaceous glands. Dandruff may be recognized as a mild form of Seborrheic dermatitis affecting only the skin of the scalp and which is distinct from simple dandruff by the presence of erythema and a greater degree of scaling with occasional itching and burning, and by the occurrence of eczematous changes to other body sites. Contrarily, simple dandruff appears (dry scales) as scaling on the scalp, but without erythema. Dandruff and seborrheic dermatitis are more than superficial stratum corneum disorders like dry scalp flaking, and include alteration of the epidermis with hyperproliferation, excess lipids, interdigitation of the corneal envelope, and parakeratosis.

Accordingly, another embodiment of the invention features uses and dermatological compositions comprising GMCY or an analogue thereof for the treatment of dandruff of the scalp.

Still other embodiments of the invention features uses and dermatological compositions comprising GMCY or an analogue thereof for the treatment of alopecia areata, atopic dermatitis, lichen simplex, photodermatoses, prurigo nodularis, psoriasis, rosacea, shingles, sunburn, urticaria (e.g. cold and heat or stress induced urticaria) and cutaneous warts.

GMCY or an analogue thereof is preferably dosed in a therapeutically relevant dose capable of alleviating the symptoms of cutaneous neurogenic inflammation, such as alleviating at least pruritus, but also to some extent erythema, dry skin and/or scaling in a subject suffering from or diagnosed with a disease mentioned herein. The therapeutically relevant dose varies according to the formulation technique, choice of vehicle as well as to the type of skin disorder and to the genetic race of the subject administering the formulation. According to this invention there is required a minimum dose in order to achieve sufficient anti-neurogenic-inflammatory effect. Therefore, in current interesting embodiments of the invention, uses and dermatological composition comprises a minimum dose of GMCY or an analogue thereof corresponding to an amount of 0.05% of GMCY or an analogue thereof by weight of the dermatological composition, such as a minimum dose of 0.1%, 0.15%, 0.2%, 0.25%, 0.3%, 0.35%, 0.4%, 0.45% and 0.5% of GMCY or an analogue thereof by weight of the dermatological composition.

Furthermore, according to this invention it has been found that GMCY or an analogue thereof causes burning or weak irritation in higher doses. Therefore, in current interesting embodiments of the invention, the dermatological composition comprises for reasons of safety and convenience a maximal dose of GMCY or an analogue thereof in an amount of 5% of GMCY or an analogue thereof by weight of the dermatological composition, such as in a maximum doses of 4.5%, 4%, 3.5%, 3%, 2.5%, 2%, 1.5%, 1.25 1%, 0.8, 0.6 and 0.5% of GMCY or an analogue thereof by weight of the dermatological composition.

Therefore, in current interesting embodiments of the invention, the dermatological composition comprises GMCY or an analogue thereof in an amount ranging between 0.05 and 5% of GMCY or an analogue thereof by weight of a dermatological composition, such as specifically in an amount ranging between 0.05 and 4.5, 0.05 and 4, 0.05 and 3.5, 0.05 and 3, 0.05 and 2.5, 0.05 and 2, 0.05 and 1.5, 0.05 and 1.25, 0.05 and 1, 0.05 and 0.8, or 0.05 and 0.6 % of GMCY or an analogue thereof by weight of the dermatological composition.

In other interesting embodiments of the invention, the dermatological composition comprises GMCY or an analogue thereof in an amount ranging between 0.1 and 5% by weight of the dermatological composition, such as between 0.1 and 4.5, 0.1 and 4, 0.1 and 3.5, 0.1 and 3, 0.1 and 2.5, 0.1 and 2, 0.1 and 1.5, 0.1 and 1.25, 0.1 and 1, 0.1 and 0.8, or 0.1 and 0.6 by weight of the dermatological composition.

In other interesting embodiments of the invention, the dermatological composition comprises GMCY or an analogue thereof in an amount ranging between 0.15 and 5% by weight of the dermatological composition, such as between 0.15 and 4.5, 0.15 and 4, 0.15 and 3.5, 0.15 and 3, 0.15 and 2.5, 0.15 and 2, 0.15 and 1.5, 0.15 and 1.25, 0.15 and 1, 0.15 and 0.8, or 0.15 and 0.6 by weight of the dermatological composition.

In still other interesting embodiments of the invention, the dermatological composition comprises GMCY or an analogue thereof in an amount ranging between 0.2 and 5% by weight of the dermatological composition, such as between 0.2 and 4.5, 0.2 and 4, 0.2 and 3.5, 0.2 and 3, 0.2 and 2.5, 0.2 and 2, 0.2 and 1.5, 0.2 and 1.25, 0.2 and 1, 0.2 and 0.8, or 0.2 and 0.6 by weight of the dermatological composition.

In still more interesting embodiments of the invention, the dermatological composition comprises GMCY or an analogue thereof in an amount of about 0.1% by weight of the dermatological composition, such as 0.2, 0.25, 0.3, 0.35, 0.4, 0.45, 0.5, 0.55, 0.6, 0.65, 0.7, 0.75, 0.8, 0.9 and 1% by weight of the dermatological composition.

As mentioned, the invention provides use of GMCY as the therapeutically active ingredient in the treatment of cutaneous neurogenic inflammation because it has now been discovered that GMCY has sufficient anti-inflammatory activity on its own without being combined with another anti-inflammatory agent. That is to say that GMCY or an analogue thereof can be applied as the primary therapeutically active ingredient or even as the sole therapeutically active ingredient in the treatment of skin conditions or skin disorders defined herein.

Therefore, in some embodiments of the invention, the dermatological composition comprises GMCY or an analogue thereof as the sole therapeutically active ingredient in the treatment of skin conditions or skin disorders associated with cutaneous neurogenic inflammation.

In other embodiments, the invention features uses of 1-glyceryl monocaprylate or an analogue thereof, such as uses of a dermatological compositions comprising 1-glyceryl monocaprylate or an analogue thereof, in the treatment of cutaneous neurogenic inflammation, such as the treatment of a skin condition or skin disorder associated with cutaneous neurogenic inflammation, with the proviso that the uses and dermatological composition described herein does not specifically comprise nicotinamide, thioniacinamide, N2-methyl-niacinamide, N2-ethyl-niacinamide and aminoniacinamide or in some instances even a pyridine carboxy derivative defined in WO 2004/000333.

In another wording, such embodiments are directed to a method for the treatment of neurogenic inflammation in skin, comprising administering a therapeutically effective amount of 1-glyceryl monocaprylate or an analogue thereof to the affected skin area of a subject in need thereof, with the proviso that the method of treatment or dermatological composition described herein does not specifically comprise nicotinamide, thioniacinamide, N2-methyl-niacinamide, N2-ethyl-niacinamide and aminoniacinamide or in some instances even a pyridine carboxy derivative defined in WO 2004/000333.

A pyridine carboxy derivative defined in WO 2004/000333 encompass the following generic formula II; 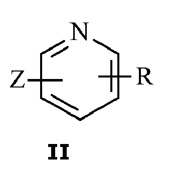 wherein R is one substituent selected from the group consisting of -C(=X)Rₐ" and - CH(R_{b}")XH;
wherein X is O or S,
Rₐ" is selected from the group consisting of H, OH, OR"', NH₂, NHR"', NR"'R"", CH₂COOH, O⁻Y⁺ and halogen,
R_{b}'' is selected from the group consisting of H and CH₂COOH,
wherein R''' and R'''' are independently selected from H, OH, , optionally substituted C₁-C₂₀ straight-chain, branched or cyclic alkyl, optionally containing one or more multiple bonds, and aryl,and wherein Y⁺ is a cation selected from optionally substituted mono-, di-, tri- or tetraalkylammonium ions, ammonium ion, and alkali metal ions,;
Z is present 0, 1, 2, 3, or 4 times and selected from the group consisting of hydrogen, halogen, NH₂, methyl, OR'" or -SH,
and wherein the pyridine carboxy derivative may optionally be substituted. The term "optionally substituted" is intended to mean the substitution of one or more hydrogen atoms is substituted with another atom, chemical group or entity, termed substituents. Illustrative examples of substituents include carboxyl, formyl, amino, hydroxyl, halogen, nitro, sulphono, sulphanyl, C1-6-alkyl, aryl, aryloxy, aryloxycarbonyl, arylcarbonyl, heteroaryl, amino, mono- and di(C1-6-alkyl)amino; carbamoyl, mono- and di(C1-6-alkyl)aminocarbonyl, amino-C1-6-alkyl-aminocarbonyl, mono- and di(C1-6-alkyl)amino-C1-6-alkyl-aminocarbonyl, C1-6-alkylcarbonylamino, cyano, guanidino, carbamido, C1-6-alkanoyloxy, C1-6-alkylsulphonyloxy, dihalogen-C1-6-alkyl, trihalogen-C1-6-alkyl, C1-6-alkoxyl, oxo, C1-6-carboxyl, C1-6-alkoxycarbonyl, C1-6-alkylcarbonyl,
where aryl and heteroaryl representing substituents may be substituted 1-5 times with C1-6-alkyl, C1-6-alkoxy, nitro, cyano, hydroxy, amino or halogen. In general, the above substituents may be susceptible to further optional substitution.

The term "C₁-C₂₀ alkyl" is intended to mean a linear or branched saturated hydrocarbon chain wherein the longest chains has from one to twenty carbon atoms, such as methyl, ethyl, n-propyl, isopropyl, n-butyl, isobutyl, sec-butyl, tert-butyl, pentyl, isopentyl, neopentyl, hexyl, heptyl, octyl, undecacyl, dodecyl, etc. A branched hydrocarbon chain is intended to mean a C₁-C₂₀ alkyl substituted at any carbon with a hydrocarbon chain. The C₁-C₂₀ alkyl chain of the present invention may be optionally substituted.

The term "C₂-C₂₀ alkenyl" is intended to mean a linear or branched unsaturated hydrocarbon chain with one or more double bindings and wherein the longest chains has from one to twenty carbon atoms. A branched hydrocarbon chain is intended to mean a C₁-C₂₀ alkyl substituted at any carbon with a hydrocarbon chain. The C₂-C₂₀ alkenyl chain of the present invention may be optionally substituted.

The term "C₁-C₂₀ alkoxyl" is intended to mean a linear or branched hydrocarbon chain wherein the longest chains has from one to twenty carbon atoms, such as methoxy, ethoxyl, n-propoxyl, isopropoxyl, n-butoxyl, isobutoxyl, isopentoxyl, hexoxyl, heptoxyl, octoxyl, etc. A branched hydrocarbon chain is intended to mean a C₁-C₂₀ alkyl substituted at any carbon with a hydrocarbon chain. The C₁-C₂₀ alkyl chain of the present invention may be optionally substituted.

The term "halogen" includes fluorine, chlorine, bromine and iodine.

Specifically, the a pyridine carboxy derivative as defined in WO 2004/000333 is a pyridine-3-carboxy derivative such as a pyridine carboxy derivative selected from the group consisting of niacinamide, thioniacinamide, 6-aminoniacinamide, N2-methylniacinamide, N2-ethylniacinamide, nicotinic acid, 6-methoxy-niacinamide and salts thereof.

Therefore, in various uses and dermatological compositions of this invention, the dermatological composition described herein does not specifically comprise a pyridine carboxy derivative selected from the group consisting of niacinamide, thioniacinamide, 6-aminoniacinamide, N2-methylniacinamide, N2-ethylniacinamide, nicotinic acid, 6-methoxy-niacinamide and salts thereof. More preferably, the dermatological composition described herein does not specifically comprise a pyridine carboxy derivative selected from the group consisting of nicotinamide, thioniacinamide, N2-methyl-niacinamide, N2-ethyl-niacinamide and aminoniacinamide and salts thereof.

Specifically it can be mentioned that where the skin disorder is psoriasis, pruritus, urticaria, atopic eczema, contact dermatitis, seborrhoeic dermatitis, acne, rosacea, alopecia, or insect bite, uses or dermatological composition described herein does not specifically comprise nicotinamide, thioniacinamide, N2-methyl-niacinamide, N2-ethyl-niacinamide and aminoniacinamide or in some instances even a pyridine carboxy derivative defined in WO 2004/000333.

In some instances, the GMCY or an analogue thereof are combined with other treatment agents that are well known in the treatment of skin conditions and skin disorders defined herein.

Accordingly, in some embodiments of the invention, GMCY or an analogue thereof is combined with or co-administered with one or more another treatment agent usually applied in the treatment of pruritus, seborrheic dermatitis, dandruff of the scalp, alopecia areata, atopic dermatitis, dry skin, herpes zoster infection, lichen simplex, prurigo nodularis, psoriasis, photodermatoses, rosacea, sunburn and urticaria.

Such treatment agents may be selected from an agent selected from an anti-fungal agent, a corticosteroid, an NSAID, a vitamin D₃ analogue, an immunomodulating agent, an antiseptic agent, an antiviral agent, sunscreen agent or an anti-acne agent.

Typical examples of anti-fungal agents for use in the present invention are azole antifungals, including but not limited to, miconazole, econazole, terconazole, saperconazole, itraconazole, butaconazole, clotrimazole, tioconazole, fluconazole and ketoconazole, vericonazole, fenticonazole, sertaconazole, posaconazole, bifonazole, oxiconazole, sulconazole, elubiol, vorconazole, isoconazole, flutrimazole and their pharmaceutically acceptable salts and the like. Other antifungal agents may include an allylamine or one from other chemical families, including but not limited to, ternafine, naftifine, amorolfine, butenafine, ciclopirox, griseofulvin, undecyclenic acid, haloprogin, tolnaftate, nystatin, iodine, rilopirox, BAY 108888, purpuromycin and their pharmaceutically acceptable salts.

Typical examples of corticosteroids for use in the present invention are Alclometasone, Beclometasone, Bendacort, Betamethasone, Budesonide, Ciclesonide, Ciprocinonide, Clobetasol, Clobetasone, Clocortolone, Cloprednol, Cortisone, Cortivazol, Deflazacort, Deprodone, Desonide, Desoximetasone, Desoxycortone, Dexamethasone, Dichlorisone, Diflorasone, Diflucortolone, Difluprednate, Fluclorolone, Flunisolide, Fluocinonide, Fluprednisolone, Fluticasone, Formocortal, Halcinonide, Halometasone, Hydrocortamate, Hydrocortisone, Isoflupredone, Loteprednol, Mazipredone, Medrysone, Meprednisone, Methylprednisolone, Mometasone, Paramethasone Acetate, Prednicarbate, Prednisolone, Prednisone, Prednylidene, Procinonide, Rimexolone, Suprarenal, Tixocortol, Triamcinolone and Ulobetasol and their pharmaceutically acceptable salts and known derivates.

Typical examples of vitamin D₃ analogue for use in the present invention are 24-Cyclopropyl-9,10-secochola-5,7,10(19),22-tetraene-1,3,24-triol (calcipotriol); 1,25-Dihydroxyvitamin D3 (calcitriol); 1,24-Dihydroxyvitamin D3 (tacalcitol), 1,25-Dihydroxy-22-oxavitamin D3 (maxacalcitol); and mixtures thereof, including hydrates thereof.

Typical examples of NSAIDs for use in the present invention are those which can be topically applied, such as ibuprofen, diclofenac, oxaprozin and indometacin.

Typical examples of anti-viral agents for use in the present invention are amantadine, rimantadin, acyclovir, famciclovir, foscarnet, ganciclovir, idoxuridine, ribavirin, sorivudine, trifluridine, valacyclovir, vidarabin, didanosine, stavudine, zalcitabine, zidovudine, interferon alpha, and edoxudine and and their pharmaceutically acceptable salts and known derivates.

Where the treatment is directed to acne, GMCY may be combined with or co-administered with benzoyl peroxide, retinoids, or anti-bacterials.

Typical examples of anti-bacterials for use in the present invention are azelaic acid, clarithromycin, clindamycin, doxycycline, erythromycin, isotretinoin, ivermectin, metronidazole, minocycline, oxytetracycline, permethrin and tetracycline,

Where the treatment is directed to photosensitivity or photodermatoses, GMCY may be combined with or co-administered with a sunscreen agent.

Where the treatment is directed to dandruff of the scalp, GMCY may be combined with or co-administered with tar, salicylic acid, pyrithione zinc, selenium sulfide, and/or sulfur.

### Dermatological compositions

Another aspect of the invention features a dermatological composition formulated for the application to skin for the local treatment of a skin disorder defined herein, the dermatological composition comprises 1-glyceryl monocaprylate or an analogue thereof as the therapeutically active ingredient and further comprises a dermatologically acceptable vehicle.

It should be understood that the dermatological compositions are meant to be administered to a portion of the skin, such as to the portion of skin affected by cutaneous neurogenic inflammation, such as to the portion of skin affected by a skin condition or skin disorder defined herein, for the local treatment of such inflammation, conditions or disorders.

The phrase "a dermatologically acceptable vehicle" is meant to define a vehicle in liquid, semi-solid or solid form, for example a vehicle / dermatological composition formulated as a cream, an ointment, a gel, a liniment, a foam, a powder, a solution, a spray-on-solution, a paste, a shampoo, or they may be provided in combination with a "finite" carrier, for example a non-spreading material that retains its form, including, for example, a patch, bioadhesive, dressing or bandage. Furthermore, in accordance with current formulation practice, the 1-glyceryl monocaprylate or an analogue thereof may be formulated in aqueous or non-aqueous vehicles, or mixtures thereof, such as in the form of a solution, emulsion, dispersion, suspension or ointment comprising the 1-glyceryl monocaprylate or an analogue thereof.

Guidance for the preparation of dermatological compositions of the invention can be found in "Remington: The science and practice of pharmacy" 20th ed. Mack Publishing, Easton PA, 2000 ISBN 0-912734-04-3 and "Encyclopaedia of Pharmaceutical Technology", edited by Swarbrick, J. & J. C. Boylan, Marcel Dekker, Inc., New York, 1988 ISBN 0-8247-2800-9 or a newer edition. As well known to the skilled person, illustrative additives to dermatological compositions include, but is not limited to: ointment bases, solvents, buffering agents, pH-adjusting agents, preservatives, humectants, chelating agents, antioxidants, stabilizers, emulsifying agents, suspending agents, gel-forming agents, perfumes, skin protective agents.

For use in the present invention, the dermatological composition preferably comprises a dose, such as an anti-neurogenic inflammatory dose, of GMCY or an analogue thereof in an amount ranging between 0.05 and 5% by weight of the dermatological composition, such as specifically in an amount ranging between 0.05 and 4.5, 0.05 and 4, 0.05 and 3.5, 0.05 and 3, 0.05 and 2.5, 0.05 and 2, 0.05 and 1.5, 0.05 and 1, 0.05 and 0.8, or 0.05 and 0.6 % by weight of the dermatological composition.

In other interesting embodiments of the invention, the dermatological composition comprises GMCY or an analogue thereof in an amount ranging between 0.1 and 5% by weight of the dermatological composition, such as between 0.1 and 4.5, 0.1 and 4, 0.1 and 3.5, 0.1 and 3, 0.1 and 2.5, 0.1 and 2, 0.1 and 1.5, 0.1 and 1.25, 0.1 and 1, 0.1 and 0.8, or 0.1 and 0.6 by weight of the dermatological composition.

In other interesting embodiments of the invention, the dermatological composition comprises GMCY or an analogue thereof in an amount ranging between 0.15 and 5% by weight of the dermatological composition, such as between 0.15 and 4.5, 0.15 and 4, 0.15 and 3.5, 0.15 and 3, 0.15 and 2.5, 0.15 and 2, 0.15 and 1.5, 0.15 and 1.25, 0.15 and 1, 0.15 and 0.8, or 0.15 and 0.6 by weight of the dermatological composition.

In other interesting embodiments of the invention, the amount of GMCY or an analogue thereof ranges between 0.2 and 5% by weight of the dermatological composition, such as between 0.2 and 4.5, 0.2 and 4, 0.2 and 3.5, 0.2 and 3, 0.2 and 2.5, 0.2 and 2, 0.2 and 1.5, 0.2 and 1, 0.2 and 0.8, or 0.2 and 0.6 by weight of the dermatological composition.

In still more interesting embodiments of the invention, the dermatological composition comprises GMCY or an analogue thereof in an amount of about 0.1% by weight of the dermatological composition, such as 0.2, 0.25, 0.3, 0.35, 0.4, 0.45, 0.5, 0.55, 0.6, 0.65, 0.7, 0.75, 0.8, 0.9 and 1% by weight of the dermatological composition.

Furthermore, for use in the present invention, the dermatological composition may be physically and chemically stable. Therefore, the dermatological composition preferably comprises a dermatologically acceptable vehicle with pH-values close to neutral, such as pH values in the range between 5.5 and 8.5, more preferably between 6 and 8, even more preferably between 6.5 and 7.5, such as about 7.

As mentioned above, in some embodiments of the invention, the dermatological composition comprises GMCY or an analogue thereof as the primary therapeutically active ingredient and even as sole therapeutically active ingredient.

In other embodiments, the dermatological composition described herein does not specifically comprise nicotinamide or in some instances even a pyridine carboxy derivative, such as those defined in WO 2004/000333. Preferably, a dermatological composition does not specifically comprise a pyridine carboxy derivative selected from the group consisting of niacinamide, thioniacinamide, 6-aminoniacinamide, N2-methylniacinamide, N2-ethylniacinamide, nicotinic acid, 6-methoxy-niacinamide and salts thereof.

In still other embodiments of the invention, the dermatological composition further comprises one or more treatment agent usually applied in the treatment of pruritus, seborrheic dermatitis, dandruff of the scalp, alopecia areata, atopic dermatitis, dry skin, herpes zoster infection, lichen simplex, prurigo nodularis, psoriasis, photodermatoses, rosacea, sunburn, urticaria, and wound healing.

Therefore, a dermatological composition of this invention may further comprise one or more treatment agents selected from an anti-fungal agent, a corticosteroid, an NSAID, a vitamin D3 analogue, an immunomodulating agent, an antiseptic agent, an antiviral agent, sunscreen agent or an anti-acne agent.

Typical examples of anti-fungal agents for use in the present invention are azole antifungals, including but not limited to, miconazole, econazole, terconazole, saperconazole, itraconazole, butaconazole, clotrimazole, tioconazole, fluconazole and ketoconazole, vericonazole, fenticonazole, sertaconazole, posaconazole, bifonazole, oxiconazole, sulconazole, elubiol, vorconazole, isoconazole, flutrimazole and their pharmaceutically acceptable salts and the like. Other antifungal agents may include an allylamine or one from other chemical families, including but not limited to, ternafine, naftifine, amorolfine, butenafine, ciclopirox, griseofulvin, undecyclenic acid, haloprogin, tolnaftate, nystatin, iodine, rilopirox, BAY 108888, purpuromycin and their pharmaceutically acceptable salts.

Typical examples of corticosteroids for use in the present invention are Alclometasone, Beclometasone, Bendacort, Betamethasone, Budesonide, Ciclesonide, Ciprocinonide, Clobetasol, Clobetasone, Clocortolone, Cloprednol, Cortisone, Cortivazol, Deflazacort, Deprodone, Desonide, Desoximetasone, Desoxycortone, Dexamethasone, Dichlorisone, Diflorasone, Diflucortolone, Difluprednate, Fluclorolone, Flunisolide, Fluocinonide, Fluprednisolone, Fluticasone, Formocortal, Halcinonide, Halometasone, Hydrocortamate, Hydrocortisone, Isoflupredone, Loteprednol, Mazipredone, Medrysone, Meprednisone, Methylprednisolone, Mometasone, Paramethasone Acetate, Prednicarbate, Prednisolone, Prednisone, Prednylidene, Procinonide, Rimexolone, Suprarenal, Tixocortol, Triamcinolone and Ulobetasol and their pharmaceutically acceptable salts and known derivates.

Typical examples of vitamin D₃ analogue for use in the present invention are 24-Cyclopropyl-9,10-secochola-5,7,10(19),22-tetraene-1,3,24-triol (calcipotriol); 1,25-Dihydroxyvitamin D3 (calcitriol); 1,24-Dihydroxyvitamin D3 (tacalcitol), 1,25-Dihydroxy-22-oxavitamin D3 (maxacalcitol); and mixtures thereof, including hydrates thereof.

Typical examples of NSAIDs for use in the present invention are those which can be topically applied, such as ibuprofen, diclofenac, oxaprozin and indometacin.

Typical examples of anti-viral agents for use in the present invention are amantadine, rimantadin, acyclovir, famciclovir, foscarnet, ganciclovir, idoxuridine, ribavirin, sorivudine, trifluridine, valacyclovir, vidarabin, didanosine, stavudine, zalcitabine, zidovudine, interferon alpha, and edoxudine and and their pharmaceutically acceptable salts and known derivates.

Where the treatment is directed to acne, GMCY may be combined with or co-administered with benzoyl peroxide, retinoids, or anti-bacterials.

Typical examples of anti-bacterials for use in the present invention are azelaic acid, clarithromycin, clindamycin, doxycycline, erythromycin, isotretinoin, ivermectin, metronidazole, minocycline, oxytetracycline, permethrin and tetracycline,

Where the treatment is directed to photosensitivity or photodermatoses, GMCY may be combined with or co-administered with a sunscreen agent.

Where the treatment is directed to dandruff of the scalp, GMCY may be combined with or co-administered with tar, salicylic acid, pyrithione zinc, selenium sulfide, and/or sulfur.

### Examples

### Example 1

Effect on capsaicin-induced neurogenic inflammation in mice and binding to the TRP vanillioid receptor.

The mechanism of cutaneous neurogenic inflammation is connected with the release of neuropeptides from sensory nerve endings. The neuropeptide substance P is believed to play a major role in inducing an inflammatory response by affecting different inflammatory cells. The capsaicin ear oedema model is based on the ability of capsaicin (8-methyl_N-vanillyl-6-nonenamide) to activate the Vanilloid receptor (TRPV1 receptor), which leads to an increased level of intracellular calcium and release of substance P from sensory neurons *(*Richardson JD, Vasko MR. Cellular mechanisms of neurogenic inflammation. J Pharmacol Exp Ther 2002 Sep;302(3):839-45*).*

In this study various concentrations of 1-glyceryl monocaprylate (GMCY) obtained from or 1-glyceryl monocaprate (commercially available) (2000µg/ear, 450µg/ear and 100µg/ear dissolved in ethanol 96%) were topically co-administered with capsaicin (250µg/ear dissolved in ethanol 96%) to NMRI mice (4-5 weeks old obtained from Taconic, DK-4623 Lille Skensved.) and the ear swelling was measured 30 minutes later. In 4 hours a second application of capsaicin was applied and the ear swelling measured 15 minutes later.

Results of ear swelling after the second capsaicin application are shown in the table 1.

**Table 1.**

| **Group** | **Drug** | **Dose, µg/ear** | **% relative ear oedema (2. app)** | **% inhibition of ear oedema** |
|---|---|---|---|---|
| 1 | Negative control (Vehicle) | - | 23,4 | - |
| 2 | GMCY | 2000 µg/ear | 15,0 | 36,0 |
| 3 | GMCY | 450 µg/ear | 15,3 | 34,4 |
| 4 | GMCY | 100 µg/ear | 15,8 | 32,6 |

Following the "first application", capsaicin-induced ear inflammation was induced both in the vehicle group and in the group with co-administering of GMCY. However, after the repeated application of capsaicin, the inflammation which could be produced in the GMCY-treated group was significantly lower than in the vehicle group in that the inflammation was reduced with about 35% in comparison to the vehicle control. All of the groups treated with 1-glyceryl monocaprate had an ear oedema similar to the vehicle treated control group indicating no inhibitory effect on neurogenic inflammation.

These results indicate that GMCY has anti-inflammatory effects on capsaicin-induced neurogenic skin inflammation in mice, probably due to depletion of substance P localized in sensory neurones. Furthermore the study indicates that esters with a longer chain length than GMCY are not effective since 1-glyceryl monocaprate had no effect.

It has been shown that GMCY does not bind to the vanilloid receptors as measured in an *in vitro* radioligand binding assay commercially available at MDS Pharma Services. The tissue used in this assay was rat vas deferens, field stimulated, and the control was 1µM of capsaicin which has an EC₅₀ of 0.76 µM. Literature reference to this assay is Wardle, KA et al. J PharmPharmacol. 48:285, 1996.

### Example 2

### Inhibition of FAAH

Fatty acid amid hydrolase (FAAH) is an integral membrane protein that hydrolyzes fatty acid amides including the endocannabinoid anandamide and N-acyl ethanolamines such as N-oleoyl ethanolamine to free fatty acids and ethanolamine.

GMCY was shown to have inhibitory effect on FAAH as demonstrated in an in vitro enzyme assay commerciable available by MDS Pharma Services. It proved to have a significant inhibitory effect on the FAAH function with an IC₅₀% of 53 µM, which is a dose that can be achieved following administration to skin.

The assay is conducted using the following test conditions; source is rat brain, substrate is Anandamide + [3H]Anandamide , the reation is[3H]Anandamide -> [3H]Ethanolamine + Arachidonic acid and the quantitation is performed on [3H]Ethanolamine using a test concentration usually of about 10µM. Oleyl Trifluromethyl Ketone can be used as the reference compound (IC50% of 0.029 µM)

### Example 3

### Inhibition of Pro-inflammatory cytokines (TNF-a and IL-1)

The ability of GMCY to inhibit release of pro-inflammatory cytokines (TNF-a and IL-1) in LPS-activated human peripheral blood mononuclear cells (PBMC) has been investigated.

GMCY was found to dose dependently inhibit bacterial lipopolysaccharide (LPS) induced release TNF-a, IL-6, and IL-1β at physiologically relevant concentrations (studies MDS062481, and AT3138). The cytokine levels were measured after stimulation with LPS and co-incubation with GMCY and compared with levels from control cells (vehicle incubation) and cells treated with the steroid Dexamethasone (figure 1).

| **Control Samples** | **IL-1β** | **TNF-α** |
|---|---|---|
| Stimulation control | 575,3 | 238,3 |
| Non-Stimulation | 6,8 | 6,8 |
| Dexamethasone control | 172 | 87 |
| *rac*-1-glycerolmonocaprylate - 10000µM | 8,2 | 6,9 |
| *rac*-1-glycerolmonocaprylate 1000µM | 124,5 | 38,5 |
| *rac*-1-glycerolmonocaprylate 100µM | 238,6 | 73,7 |
| rac-1-glycerolmonocaprylate 10µM | 320,9 | 105 |
| *rac*-1-glycerolmonocaprylate 1µM | 459,2 | 230,2 |

### Example 4

### Effect of GMCY and glyceryl fatty acid esters of higher acid chain length on PPARα agonism.

It was shown that GMCY did not act as an agonist for PPARα, but that glyceryl fatty acid esters made of higher number of acid carbon chain length (C10, C12, C14 and C16) act as activators of PPARα in that the longer the chain length, the more activation of PPARα was observed.

Agonism of PPARα can be tested by commercially available assays such as by Indigo Biosciences). The test substance is dissolved in DMSO and serial dilutions in DMSO were prepared. A plasmid was formed by fusing a ligand-binding domain of human PPARα, to a DAN-binding domain of the yest transcription factor Gal4 under the control of the SV40 promoter. This plasmid also encoded the UAS-firefly luciferase reporter under the control of the Gal4 DNA response element. Cell-cultures and transactivation assays were HEK 293-T fibroblast (ATCC Manassas, VA) which were cultured in high-glucose Dulbecco's Minimal Essential Medium (DMEM) supplemented with 10% fetal bovine serum (FBS, Sigma), 0.2 mg/ml streptomycin and 200 U/ml penicillin (Gibco Grand Island, NY). Cells were transfected with plasmid DNA using Lipofectamine reagent (In Vitrogen, Carlsbad, CA) using the manufacturers recommended procedures. Sixteen hours after treatment, the cells were lysed with passive lysis buffer (Promega, Madison, WI) and a Tecan GeniousPro (Research Triangle Park, NC). The fold induction of normalised luciferase activity was calculated relative to DMSO, DMSO-treated cells.

### Example 5

### Inhibition of Protein tyrosine kinase Syk

1-glyceryl monocaprylate inhibits the Protein tyrosine kinase enzyme Syk with an IC₅₀% value of 61 µm (Test performed at MDS Pharma, assay no 174200).

## Claims

1. Use of a dermatological composition comprising 1-glyceryl monocaprylate or an analogue thereof for the treatment of cutaneous neurogenic inflammation.

2. Use of a dermatological composition comprising 1-glyceryl monocaprylate or an analogue thereof for the treatment of a skin condition or skin disorder associated with cutaneous neurogenic inflammation in a subject in need thereof.

3. The use according to claim 2, wherein the skin condition associated with cutaneous neurogenic inflammation is pruritus.

4. The use according to claim 3, wherein pruritus is caused by or associated with a systemic disease selected from the group consisting of diabetes mellitus, hyperthyroidism, hypothyroidism, thyrotoxicosis, kidney failure, uraemic itching, liver cholestasis, primary biliary cirrhosis, polycythaemia vera, Hodgkin's disease, food allergy, leukaemia, brain tumour and adenocarcinoma.

5. The use according to claim 2, wherein the skin disorder associated with cutaneous neurogenic inflammation is a pruritic skin disorder.

6. The use according to claim 5, wherein the pruritic skin disorder is selected from the group consisting of acne, alopecia, anogenital pruritus, aquagenic pruritus, atopic dermatitis, chickenpox infection, dermatitis herpetiformis, dry skin, hemorrhoids, insect sting or bites, lichen simplex, photodermatitis, photosensitivity, pityriasis rosea, pregnancy-related dermatoses, prurigo nodularis, prurigo planus, pruritus ani, pruritic otitis, pruritic papular eruption of HIV, psoriasis, ringworm, scabies, seborrheic dermatitis, senile pruritus, shingles, urticaria, wounds and sunburn.

7. The use according to claim 2, wherein the skin disorder associated with cutaneous neurogenic inflammation is seborrheic dermatitis.

8. The use according to claim 2, wherein the skin disorder associated with cutaneous neurogenic inflammation is dandruff of the scalp.

9. The use according to claim 2, wherein the skin disorder associated with cutaneous neurogenic inflammation is alopecia areata.

10. The use according to claim 2, wherein the skin disorder associated with cutaneous neurogenic inflammation is atopic dermatitis.

11. The use according to claim 2, wherein the skin disorder associated with cutaneous neurogenic inflammation is lichen simplex.

12. The use according to claim 2, wherein the skin disorder associated with cutaneous neurogenic inflammation is photodermatoses

13. The use according to claim 2, wherein the skin disorder associated with cutaneous neurogenic inflammation is prurigo nodularis.

14. The use according to claim 2, wherein the skin disorder associated with cutaneous neurogenic inflammation is psoriasis.

15. The use according to claim 2, wherein the skin disorder associated with cutaneous neurogenic inflammation is rosacea.

16. The use according to claim 2, wherein the skin disorder associated with cutaneous neurogenic inflammation is shingles.

17. The use according to claim 2, wherein the skin disorder associated with cutaneous neurogenic inflammation is sunburn.

18. The use according to claim 2, wherein the skin disorder associated with cutaneous neurogenic inflammation is urticaria.

19. The use according to claim 2, wherein the skin disorder associated with cutaneous neurogenic inflammation is cutaneous warts.

20. The use according to any one of the preceding claims, wherein the dermatological composition comprises 1-glyceryl monocaprylate or an analogue thereof as the primary therapeutically active ingredient.

21. The use according to any one of the preceding claims, wherein the dermatological composition comprises 1-glyceryl monocaprylate or an analogue thereof as the sole therapeutically active ingredient.

22. The use according to any one of the preceding claims, with the proviso that wherein the skin disorder is psoriasis, pruritus, urticaria, atopic eczema, contact dermatitis, seborrhoeic dermatitis, acne, rosacea, alopecia, or insect bite, the dermatological composition does not comprise nicotinamide, thioniacinamide, N2-methyl-niacinamide, N2-ethyl-niacinamide and aminoniacinamide.

23. The use according to any one of claims 1 to 22, with the proviso that wherein the skin disorder is psoriasis, pruritus, urticaria, atopic eczema, contact dermatitis, seborrhoeic dermatitis, acne, rosacea, alopecia, or insect bite, the dermatological composition does not comprise niacinamide, thioniacinamide, 6-aminoniacinamide, N2-methylniacinamide, N2-ethylniacinamide, nicotinic acid, 6-methoxy-niacinamide or salts thereof.

24. The use according to any one of claims 1 to 22, with the proviso that the dermatological composition does not comprise a pyridine carboxy derivative described in the international patent application WO 2004/000333.

25. The use according to any one of the preceding claims, wherein the dermatological composition comprises 1-glyceryl monocaprylate or an analogue thereof in combination with an agent selected from the group consisting of an anti-fungal agent, a corticosteroid, an NSAID, a vitamin D₃ analogue, an immunomodulating agent, an antiseptic agent, an antiviral agent, sunscreen agent or an anti-acne agent.

26. The use according to claim 25, wherein the anti-fungal agent is selected from the group consisting of miconazole, econazole, terconazole, saperconazole, itraconazole, butaconazole, clotrimazole, tioconazole, fluconazole and ketoconazole, vericonazole, fenticonazole, sertaconazole, posaconazole, bifonazole, oxiconazole, sulconazole, elubiol, vorconazole, isoconazole and flutrimazole.

27. A dermatological composition formulated for the application to skin for the local treatment of a skin disorder, the dermatological composition comprises 1-glyceryl monocaprylate or an analogue thereof as the therapeutically active ingredient and further comprises a dermatologically acceptable vehicle.

28. The dermatological composition according to claim 27, wherein the 1-glyceryl monocaprylate or an analogue thereof is present in an amount ranging between 0.05 and 5% by weight of the dermatological composition.

29. The dermatological composition according to claim 27, wherein the dermatological composition comprises 1-glyceryl monocaprylate or an analogue thereof in an amount ranging between 0.05 and 4% weight of the dermatological composition.

30. The dermatological composition according to claim 27, wherein the dermatological composition comprises 1-glyceryl monocaprylate or an analogue thereof in an amount ranging between 0.05 and 3% weight of the dermatological composition.

31. The dermatological composition according to claim 27, wherein the dermatological composition comprises 1-glyceryl monocaprylate or an analogue thereof in an amount ranging between 0.05 and 2% weight of the dermatological composition.

32. The dermatological composition according to claim 27, wherein the dermatological composition comprises 1-glyceryl monocaprylate or an analogue thereof in an amount ranging between 0.05 and 1% weight of the dermatological composition.

33. The dermatological composition according to claim 27, wherein the dermatological composition comprises 1-glyceryl monocaprylate or an analogue thereof in an amount of 0.2% by weight.

34. The dermatological composition according to claim 27, wherein the dermatological composition comprises 1-glyceryl monocaprylate or an analogue thereof in an amount of 0.25% by weight.

35. The dermatological composition according to claim 27, wherein the dermatological composition comprises 1-glyceryl monocaprylate or an analogue thereof in a dose of 0.3% by weight.

36. The dermatological composition according to claim 27, wherein the dermatological composition comprises 1-glyceryl monocaprylate or an analogue thereof in an amount of 0.4% by weight.

37. The dermatological composition according to claim 27, wherein the dermatological composition comprises 1-glyceryl monocaprylate or an analogue thereof in an amount of 0.5% by weight.

38. The dermatological composition according to claim 27, wherein the dermatological composition comprises 1-glyceryl monocaprylate or an analogue thereof in an amount of 0.6% by weight.

39. The dermatological composition according to claim 27, wherein the dermatological composition comprises 1-glyceryl monocaprylate or an analogue thereof in an amount of 0.7% by weight.

40. The dermatological composition according to claim 27, wherein the dermatological composition comprises 1-glyceryl monocaprylate or an analogue thereof in an amount of 0.8% by weight.

41. The dermatological composition according to claim 27, wherein the dermatological composition comprises 1-glyceryl monocaprylate or an analogue thereof in an amount of 1.0 % by weight.

42. The dermatological composition according to claim 27, wherein the dermatologically acceptable vehicle has a pH in the range of between 5.5 and 8.5.

43. The dermatological composition according to claim 27, wherein the dermatologically acceptable vehicle has a pH in the range of between 6 and 8.

44. The dermatological composition according to claim 27, wherein the dermatologically acceptable vehicle has a pH in the range of between 6.5 and 7.5.

45. The dermatological composition according to claim 27, wherein the 1-glyceryl monocaprylate or an analogue thereof is present in an amount ranging between 0.05 and 5% by weight of the dermatological composition and the dermatologically acceptable vehicle has a pH in the range of between 5.5 and 8.5.

46. The dermatological composition according to claim 27, wherein the 1-glyceryl monocaprylate or an analogue thereof is present in an amount ranging between 0.05 and 5% by weight of the dermatological composition and the dermatologically acceptable vehicle has a pH in the range of between 6 and 8.

47. The dermatological composition according to claim 27, wherein the 1-glyceryl monocaprylate or an analogue thereof is present in an amount ranging between 0.05 and 5% by weight of the dermatological composition and the dermatologically acceptable vehicle has a pH in the range of between 6.5 and 7.5.

48. The dermatological composition according to claim 27, wherein the 1-glyceryl monocaprylate or an analogue thereof is present in an amount ranging between 0.05 and 3% by weight of the dermatological composition and the dermatologically acceptable vehicle has a pH in the range of between 5.5 and 8.5.

49. The dermatological composition according to claim 27, wherein the 1-glyceryl monocaprylate or an analogue thereof is present in an amount ranging between 0.05 and 3% by weight of the dermatological composition and the dermatologically acceptable vehicle has a pH in the range of between 6 and 8.

50. The dermatological composition according to claim 27, wherein the 1-glyceryl monocaprylate or an analogue thereof is present in an amount ranging between 0.05 and 2% by weight of the dermatological composition and the dermatologically acceptable vehicle has a pH in the range of between 5.5 and 8.5.

51. The dermatological composition according to claim 27, wherein the 1-glyceryl monocaprylate or an analogue thereof is present in an amount ranging between 0.05 and 2% by weight of the dermatological composition and the dermatologically acceptable vehicle has a pH in the range of between 6 and 8.

52. The dermatological composition according to claim 27, wherein the dermatologically acceptable vehicle is in liquid form.

53. The dermatological composition according to claim 27, wherein the dermatologically acceptable vehicle is in semi-solid form.

54. The dermatological composition according to claim 27, wherein the dermatologically acceptable vehicle is formulated as an emulsion.

55. The dermatological composition according to claim 27, wherein the dermatologically acceptable vehicle is formulated as a gel.

56. The dermatological composition according to claim 27, wherein the dermatologically acceptable vehicle is formulated as a foam.

57. The dermatological composition according to claim 27, wherein the dermatologically acceptable vehicle is formulated as a shampoo.

58. The dermatological composition according to claim 27, wherein the 1-glyceryl monocaprylate or an analogue thereof is the sole therapeutically active ingredient.

59. The dermatological composition according to claim 27, with the proviso that the dermatological composition does not comprise a pyridine carboxy derivative selected from the group consisting of niacinamide, thioniacinamide, 6-aminoniacinamide, N2-methylniacinamide, N2-ethylniacinamide, nicotinic acid, 6-methoxy-niacinamide and salts thereof.

60. The dermatological composition according to claim 27, with the proviso that the dermatological composition does not comprise a pyridine carboxy derivative as defined in WO 2004/000333.

61. The dermatological composition according to claim 27, further comprising one or more treatment agents selected from an anti-fungal agent, a corticosteroid, an NSAID, a vitamin D₃ analogue, an immunomodulating agent, an antiseptic agent, an antiviral agent, sunscreen agent and an anti-acne agent.

62. The dermatological composition according to claim 61, wherein the anti-fungal agent is selected from the group consisting of miconazole, econazole, terconazole, saperconazole, itraconazole, butaconazole, clotrimazole, tioconazole, fluconazole and ketoconazole, vericonazole, fenticonazole, sertaconazole, posaconazole, bifonazole, oxiconazole, sulconazole, elubiol, vorconazole, isoconazole, flutrimazole, allylamine, ternafine, naftifine, amorolfine, butenafine, ciclopirox, griseofulvin, undecyclenic acid, haloprogin, tolnaftate, nystatin, iodine, rilopirox, BAY 108888, purpuromycin and their pharmaceutically acceptable salts.
